# EUROPÄISCHE PATENTANMELDUNG

(11) **EP 1 300 387 A1**
(43) Veröffentlichungstag der Anmeldung: **09.04.2003**
(21) Anmeldenummer: 01123859.9
(22) Anmeldetag: 05.10.2001
(51) Int. Cl.: C07C 67/03, C07C 69/66, C07C 69/68

(54) **Verfahren zum Herstellen von Estern einer Hydroxysäure**

(71) Anmelder: Haltermann GmbH, 20095 Hamburg (DE)
(72) Erfinder: Hildebrandt, Rainer, 21149 Hamburg (DE); Vollmer, Hans-Jürgen, Dr., 22149 Hamburg (DE); Alscher, Arnold, Dr., 22391 Hamburg (DE); Höltmann, Wilhelm, Dr., 21220 Seevetal (DE)
(74) Vertreter: Glawe, Delfs, Moll & Partner

(57) **Zusammenfassung**

Gegenstand der Erfindung ist ein Verfahren zum Herstellen von Estern einer Hydroxysäure und eines C₁-C₈-Alkohols. Erfindungsgemäß wird die Veresterung durch Reaktivdestillation an einem heterogenen Katalysator vorgenommen. Die Erfindung erlaubt insbesondere die Herstellung von Milchsäureestern hoher Reinheit mit guter Ausbeute.

## Beschreibung

Die Erfindung betrifft ein Verfahren zum Herstellen von Estern einer Hydroxysäure und eines C₁-C₈-Alkohols, insbesondere zur Herstellung von Lactaten.

Milchsäureester (nachfolgend auch Lactate genannt) eignen sich als Lösungsmittel für Cellulosenitrate, -acetate, und -ether, Chlorkautschuk, Polyvinylverbindungen und dergleichen. Sie werden ferner als Weichmacher für Cellulose und Vinylharze, als Lacklösemittel sowie als Lösemittel bei der Chipherstellung verwendet.

Die Synthese von Milchsäureestern erfolgt üblicherweise aus Milchsäure und dem entsprechenden Alkohol in flüssiger Phase in Anwesenheit saurer Katalysatoren. Da flüssige Säurekatalysatoren in dem Endprodukt verbleiben, kommt es zu einer Vielzahl unerwünschter Nebenreaktionen.

Der Erfindung liegt die Aufgabe zugrunde, ein eingangs genanntes Verfahren zu schaffen, das eine Herstellung der genannten Ester in guter Ausbeute und mit hoher Reinheit gestattet.

Die Erfindung löst diese Aufgabe dadurch, daß die Veresterung durch Reaktivdestillation an einem heterogenen Katalysator erfolgt.

Der Begriff Reaktivdestillation bezeichnet die Kombination von chemischer Reaktion (hier Veresterung) und destillativer Stofftrennung. Entscheidend ist, daß unmittelbar im Anschluß an die katalysierte Reaktion eine destillative Stofftrennung der Produkte und ggf. verbleibender Edukte der Reaktion erfolgt. Anders als bei einer homogenen Katalyse mit einem flüssigen Katalysator wird somit das Reaktionsprodukt unmittelbar nach der Reaktion von dem heterogenen Katalysator getrennt.

Als Katalysator eignet sich jedes Material, das die Veresterungsreaktion zu katalysieren vermag. Bevorzugt werden saure Katalysatoren verwendet. Der Katalysator ist heterogen. Dies bedeutet, daß er in einem anderen Aggregatzustand vorliegt als Edukte und Produkte der Veresterungsreaktion. In der Regel wird ein Festphasenkatalysator verwendet.

Als Hydroxysäure findet bevorzugt eine α-Hydroxysäure, besonders bevorzugt Milchsäure Verwendung. Bevorzugt wird ein Ester mit einem C₂-C₈-Alkohol hergestellt, besonders bevorzugt mit einem C₂-C₄-Alkohol. Der Alkohol ist bevorzugt ein primärer oder sekundärer Alkohol, der ausgewählt sein kann aus der Gruppe bestehend aus Ethanol, n-Propanol, Isopropanol, n-Butanol, 2-Butanol und sec-Butanol.

Der Katalysator ist bevorzugt in der Destillations- bzw. Rektifikationskolonne fixiert. Bevorzugt wird im Rahmen der Erfindung eine gepackte Kolonne (Definition siehe Ullmanns Encyclopedia of Industrial Chemistry, 5. Auflage, Band B3, Seite 4-71) verwendet. Die gesamte Kolonnenpackung oder ein Teil davon kann mit einem heterogenen Katalysator versehen oder durch diesen ersetzt werden.

Als Träger zur Fixierung des Katalysators kann eine in der Kolonne befestigte strukturierte Packung verwendet werden wie beispielsweise die Packung Katapak®-S der Sulzer Chemtech AG. Diese Packung besteht aus lagenweise angeordnetem Maschendrahtgewebe, zwischen die Gewebelagen kann der Katalysator in Taschen eingelagert und dadurch fixiert werden.

Als Katalysator findet bevorzugt ein saures Ionenaustauscherharz wie beispielsweise das makroporöse saure Ionenaustauscherharz Amberlyst®15, Firma Röhm und Haas, Verwendung. Es können handelsübliche Pellets dieses Katalysators mit einem Durchmesser zwischen 0,35 und 1,2 mm verwendet werden.

Die gewünschte Kontaktzeit der Edukte in der Kolonne kann durch Variation der Destillations-/Rektifikationsparameter gesteuert werden. Das höhersiedende Edukt (in der Regel die Milchsäure) wird vorzugsweise oberhalb des Katalysators in die Destillationskolonne eingespeist, das niedrigersiedende Edukt unterhalb dieses Katalysators. Die Edukte kommen so im Gegenstrom im Bereich der Katalysatorpackung miteinander zur Reaktion. Die Reaktionsparameter können ferner beeinflußt werden durch die Wahl des Rücklaufverhältnisses der Kolonne, der Kolonnentemperatur und (davon abhängig) des Drucks in der Kolonne.

Ein besonderer Vorteil der Reaktivdestillation ist, daß bei der Veresterung entstehendes Reaktionswasser destillativ sofort abgetrennt wird und damit das Reaktionsgleichgewicht auf die Seite des Esters verschoben wird. Ferner wird auf diese Weise ein ggf. in den Edukten enthaltener Wasseranteil entfernt. Technische Milchsäure hat beispielsweise in der Regel einen Wasseranteil von etwa 20%.

Bei Bedarf kann zur Wasserabtrennung zusätzlich ein geeignetes Schleppmittel verwendet werden, mit dem in den Edukten vorhandenes Wasser sowie Reaktionswasser als Azeotrop abdestilliert. Unter Umständen kann als Schleppmittel sogar der zur Veresterung verwendete Alkohol verwendet werden. Bei der Herstellung eines Isopropylethers kann im Überschuß eingesetztes Isopropanol gleichzeitig als Schleppmittel für Wasser dienen.

Die Reaktionstemperatur am Katalysator kann bei 60 - 200°C, vorzugsweise bei 60 - 150°C, weiter vorzugsweise 70 - 100°C liegen. Diese Temperatur hängt ab von den Siedepunkten der in der Kolonne destillierenden flüssigen bzw. gasförmigen Produkte, diese können ggf. variiert werden durch Einstellen eines Über- oder Unterdrucks in der Kolonne. Eine Obergrenze der Temperatur wird ferner bestimmt durch das verwendete Katalysatormaterial. Beispielsweise sind styrolbasierte Katalysatoren nur bis etwa 95 - 100°C temperaturstabil, bei höheren Temperaturen spalten sie Schwefelsäure ab. Silicobasierte Katalysatoren sind häufig temperaturstabiler bis beispielsweise etwa 200°C. Den Druck der Kolonne wählt man in der Regel so, daß der eingesetzte Alkohol bei einer Temperatur siedet, die unterhalb einer etwaigen Zersetzungstemperatur des verwendeten heterogenen Katalysators liegt.

Der bei der Reaktion erhaltene schwerflüchtige Ester wird als Sumpfprodukt aus der Kolonne abgezogen. Bei hochsiedenen Estern kann die Sumpftemperatur unter Umständen so hoch sein, daß Zersetzungsreaktionen auftreten. In diesem Fall kann man zur Senkung der Sumpftemperatur ein niedriger siedendes Lösungsmittel in dem Sumpf schneiden, das in einem nachfolgenden Destillationsschritt entfernt werden kann.

Die erfindungsgemäße Verwendung eines heterogenen Katalysators im Rahmen einer Reaktivdestillation hat den zusätzlichen Vorteil, daß in den Edukten ggf. enthaltene Verunreinigungen von Metallionen unter Deaktivierung des Katalysators aus der Flüssigphase entfernt werden. Man erhält so hochreine Ester, die besonders vorteilhaft als Lösungsmittel für Photolacke oder dergleichen bei der Chipherstellung Verwendung finden können.

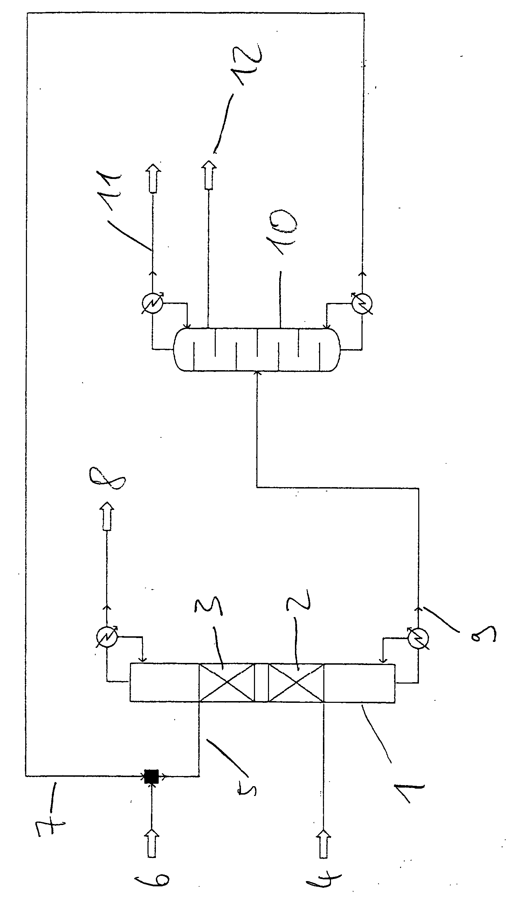

Die Erfindung wird nachfolgend anhand von Ausführungsbeispielen beschrieben. Die Figur zeigt schematisch eine Anlage zur Durchführung des erfindungsgemäßen Verfahrens.

Eine gepackte Rektifikationskolonne 1 weist mehrere Sektionen auf. Zwei mit den Bezugsziffern 2 und 3 bezeichnete Sektionen weisen eine katalytische Packung auf, es kann sich dabei um die oben erwähnte Katapak®-S der Sulzer Chemtech AG handeln. Diese katalytische Packung ist mit einem sauren Ionenaustauscherharz in Form von Pellets gefüllt.

In die Kolonne 1 wird bei 4 der Alkohol eingespeist, bei 5 erfolgt die Einspeisung von bei 6 zugeführter Milchsäure gemeinsam mit über die Leitung 7 rückgeführter Milchesäuredimere, die aus der unten erläuterten Aufarbeitung des Rohesters stammen. Aus der gepackten Kolonne 1 wird am Kolonnenkopf bei 8 Wasser (Reaktionswasser sowie ggf. in Ausgangsprodukten enthaltenes Wasser), ggf. zusammen mit einem Schleppmittel, abgezogen.

Der Rohester wird über die Leitung 9 aus dem Kolonnensumpf abgezogen. Er weist typischerweise eine Reinheit von 90 - 95% auf. Dieser Rohester wird einer destillativen Aufbereitung in einer herkömmlichen Kolonne 10 unterzogen. Aus dieser Kolonne 10 werden am Kolonnenkopf bei 11 verbleibende leicht flüchtige Anteile wie Restwasser und Alkohol abgezogen. Der Milchsäureester wird bei 12 aus der Kolonne abgezogen. Bezogen auf die eingesetzte Milchsäure erhält man ihn mit einer Ausbeute, die in der Regel über 95% liegt. Der Sumpf dieser destillativen Aufarbeitung enthält im wesentlichen als Nebenprodukt entstandene dimere Milchsäure, die über die bereits genannte Leitung 7 wieder als Edukt der gepackten Rektifikationskolonne 1 zugeführt wird. Diese problemlose Wiederverwendbarkeit dimerer Milchsäure ist ein besonderer Vorteil der vorliegenden Erfindung. Im Stand der Technik mit Flüssigkatalysatoren muß die Reaktionsmischung neutralisiert werden, um weitere Reaktionen zu unterdrücken. Diese Neutralisation macht eine anschließende sofortige Wiederverwendung (Recyklierbarkeit) aufgearbeiteter Nebenprodukte unmöglich.

Sämtliche Prozentangaben in den Beispielen sind Gewichtsprozente, soweit nicht anders angegeben.

### Beispiel 1

| Herstellung von n-Propyllactat | |
|---|---|
| Anlage | Kontinuierliche Destillationsanlage mit 5 Sektionen. Unterste Sektion und Sektion 4 und 5 mit 40 mm Durchmesser mit Sulzer CY® Packung, Sektionen 2 und 3 mit 70 mm Durchmesser und katalytischer Packung. Phasentrenner am Kopf mit Rückführung der organischen Phase über Sektion 4. Milchsäureeinsatz über Sektion 3 und Propanoleinsatz über Sektion 1. |
| Schleppmittel | 445 g n-Heptan Erstbefüllung, weiterer Zusatz nach Bedarf bei Anstieg der Kopftemperatur. |
| Rückfluß | 1 : 0,2 |
| Einsätze | |
| n-Propanol | Über 1. Schuß mit 90°C, gesteigert bis auf 230 g/h, gesamt 13448 g |
| Milchsäure | Über 3. Schuß mit 90°C, gesteigert bis auf 350 g/h, gesamt 15850 g |
| Abläufe | |
| Wasserphase | 150 - 170 g/h, gesamt 10308 g |
| Sumpf | 420 - 480 g/h, gesamt 18288 g, Restsumpf 1350g/h |
| Temperaturen | |
| Kopf | 69 - 70°C |
| über 3. Schuß | 74 - 75°C |
| über 2. Schuß | 91 - 92°C |
| über 1. Schuß | 92 - 93°C |
| Sumpf | bis 165°C, später reduziert auf 144 - 146°C |
| Wassergehalt der Wasserphase | 82 - 85% |
| Acidity im Sumpf | 0,40 - 0,81 mg KOH/g |

Als katalytische Packung wurde Katapak®-S gefüllt mit einem sauren Ionenaustauscherharz verwendet.

Als Schleppmittel für Reaktionswasser und in den Ausgangsprodukten enthaltenes Wasser wurde n-Heptan verwendet. Die verwendete Milchsäure war 80%ig mit einem Wasseranteil von 20%. Die Kolonne wurde vor dem Aufstart mit n-Propanol und Heptan gefüllt. Die Vollständigkeit der Reaktion bezogen auf das Sumpfprodukt wurde anhand der Acidity im Sumpf überprüft. Die Feedmengen (Einsätze von n-Propanol und Milchsäure) wurde so eingestellt, daß diese Acidity im Sumpf deutlich unter 1 mg KOH/g lag.

Die Sumpftemperatur stellte sich zunächst auf etwa 165°C ein, der n-Propanolgehalt im Sumpf betrug weniger als 1%. Diese hohe Sumpftemperatur begünstigt Weiter- und Nebenreaktionen des Sumpfproduktes, daher wurde etwas n-Propanol (5 - 7%) in den Sumpf geschnitten und so die Sumpftemperatur auf etwa 144 - 146°C gesenkt. Der Gehalt von n-Propyllactat im Sumpf wurde gaschromatographisch bestimmt und betrug etwa 93,5%.

Die Sümpfe der Reaktivdestillation wurden in einer Füllkörper-Kolonne aufdestilliert. Es wurden die folgenden Fraktionen erhalten:
1. 3,1% Vorlauf mit 99,5% n-Propanol
2. 0,3% Zwischenlauf mit 25% n-Propanol und 75% Propyllactat
3. 89,3% Propyllactat mit einer Reinheit von 99,75%. Die Verunreinigungen waren 0,13% n-Propanol und 0,02% Wasser. Die Acidity betrug 0,08 mg KOH/g.
4. 7,3% Restsumpf mit einer Acidity von 7,1 mg KOH/g. Der Restsumpf enthielt vorwiegend Dimere und Oligomere.

Vorlauf, Zwischenlauf und Restsumpf wurden problemlos wieder in der Reaktivdestillation eingesetzt.

Die Fertigware wurde gaschromatographisch untersucht und wies einen Gehalt von n-Propyllactat von 99,77% auf. Der n-Propanoigehalt betrug 0,13 %, der Wassergehalt 0,02% und die Acidity 0,08 mg KOH/g.

### Beispiel 2

| Herstellung von Ethyllactat | |
|---|---|
| Anlage | Kontinuierliche Destillationsanlage mit 5 Sektionen. Unterste Sektion und Sektion 4 und 5 mit 40 mm Durchmesser mit Sulzer CY® Packung, Sektionen 2 und 3 mit 70 mm Durchmesser und katalytischer Packung. Phasentrenner am Kopf mit Rückführung der organischen Phase über Sektion 4. Milchsäureeinsatz über Sektion 3 und Ethanol über Sektion 1. |
| Schleppmittel | 120 g Diisopropylether Erstbefüllung, weiterer Zusatz nach Bedarf bei Anstieg der Kopftemperatur. Später auch Zusatz von Cyclohexan als Schleppmittel. |
| Rückfluß | 1 : 0,5 |
| Einsätze | |
| Ethanol | Über 1. Schuß mit 80°C, ca. 30 g/h, gesamt 3148 g |
| Milchsäure | Über 3. Schuß mit 90°C, ca. 55 g/h, gesamt 2631 g |
| Abläufe | |
| Wasserphase | 35 g/h, gesamt 1725 g, Wassergehalt 54 - 63% |
| Sumpf | 55 g/h, gesamt 2269 g, Wassergehalt 0,1 - 0,2%, Acidity 5 - 9 mg KOH/g |
| Temperaturen | |
| Kopf | 61°C |
| über 4. Schuß | 62°C |
| über 3. Schuß | 68 - 70°C |
| über 2. Schuß | 78 - 79°C |
| über 1. Schuß | 79°C |
| Sumpf | 102 - 130°C abhängig vom Restalkohol-Gehalt |
| Wassergehalt der Wasserphase | 54 - 63% |
| Acidity im Sumpf | 5 - 9 mg KOH/g |

Vor dem Aufstart wurde die Kolonne mit Ethanol und Diisopropylether als Schleppmittel gefüllt. Die Acidity im Sumpf war etwas höher als bei der Synthese von n-Propyllactat, da bei den niedrigen Temperaturen in der Kolonne die Veresterungsreaktion nur verhältnismäßig langsam ablief. Eine Reaktivdestillation unter Überdruck bei dementsprechend erhöhter Kolonnentemperatur kann die Reaktionsgeschwindigkeit beschleunigen.
Bei einer Sumpftemperatur von etwa 130°C betrug der Ethyllactatgehalt im Sumpf 80,7% und der Ethanolgehalt 5,7%. Der Dilactidanteil lag bei 3%.

Bei der Destillation in einer Füllkörper-Kolonne wurden die folgenden Mengen erhalten:
1. 16,6% Vorlauf - Ethanol mit 12,5% Wasser
2. 2,9% Zwischenlauf - Ethanol mit 34% Wasser
3. 18,8% Ethyllactat mit einer Reinheit von 99,6% und 0,4% Wasser
4. 42,5% Ethyllactat mit 1,2% Wasser
5. 18,8% Restsumpf mit einer Acidity von 226 mg KOH/g. Der Restsumpf enthielt vorwiegend Milchsäure und Dimere.

Die Fraktionen 3 und 4 konnten ohne Zersetzung redestilliert werden. Die Redestillation ergab Ethyllactat mit kleiner Acidity und hoher Reinheit.

Der Rückstand der Aufarbeitung enthielt hohe Anteile Milchsäure und Dilactid und kann als Edukt in die Reaktivdestillationskolonne wieder eingespeist werden. Vorteilhaft ist ein Versetzen dieses Rückstandes mit etwa 10% Wasser, um die Rückspaltung des Dilactids zu Milchsäure zu erleichtern.

Bei Wiedereinsatz der anfallenden Rückstände in der geschilderten Weise beträgt die Gesamtausbeute Ethyllactat bezogen auf die eingesetzte Milchsäure etwas über 95%.

### Beispiel 3

| Herstellung von Isopropyllactat | | |
|---|---|---|
| Versuchsserie | 1 | 2 |
| | 2 katalytische Sektionen | 3 katalytische Sektionen |
| | | |
| Anlage | Kontinuierliche Destillationsanlage mit 5 oder 6 Sektionen. Unterste Sektion und Sektion 5 und 6 mit 40 mm Durchmesser mit Sulzer CY® Packung, Sektionen 2 und 3 (Versuchsserie 1) bzw. Sektion 2, 3 und 4 (Versuchsserie 2) mit 70 mm Durchmesser und katalytischer Packung. Milchsäureeinsatz über den katalytischen Sektionen und Ethanol über Sektion 1. | |
| | | |
| Druck | Normaldruck | Normaldruck |
| Differenzdruck | 2,5 mbar | 3 mbar |
| Rückfluß | 1 : 7 bis 1 : 8,5 | 1 : 7,5 bis 1 : 8 |
| Einsätze | | |
| IPA | Über 1. Schuß mit | Über 1. Schuß mit |
| | 80°C 350 g/h, | 80°C 570 g/h, |
| | gesamt 8600 g | gesamt 6780 g |
| Milchsäure | Über 3. Schuß mit | Über 4. Schuß mit |
| | 95°C 75 g/h, | 95°C 140 g/h, |
| | gesamt 1850 g | gesamt 2160 g |
| Abläufe | | |
| Kopfprodukt | 330 g/h, | 550 g/h, |
| | mit 8 - 12% Wasser, | mit 9 - 10% Wasser, |
| | gesamt 8060 g | gesamt 8010 g |
| Sumpf | 100 g/h, gesamt 1002 g, Wassergehalt <0,02%, Acidity 3 - 14 mg KOH/g | 180 g/h, gesamt 2560 g, Wassergehalt <0,02%, Acidity 10 - 17 mg KOH/g |
| Temperaturen | | |
| über 6. Schuß | - | 80°C |
| über 5. Schuß | 80°C | 81°C |
| über 4. Schuß | 81°C | 82°C |
| über 3. Schuß | 81,5°C | 82°C |
| über 2. Schuß | 82°C | 82°C |
| über 1. Schuß | 82°C | 83°C |
| Sumpf | 92 - 152°C abhängig vom Restalkohol-Gehalt | 125 - 153°C abhängig vom Restalkohol-Gehalt |

Bei der Versuchsserie 1 wurden die Kolonnensektionen 2 und 3 mit einer katalytischen Packung gefüllt, bei der Versuchsserie 2 wurde die Reaktivzone um eine Sektion verlängert. Als Schleppmittel wurde ein Isopropanolüberschuß eingesetzt. Der Isopropanol/Wasserstrom wurde über Kopf abgezogen. Die oberhalb der katalytischen Sektionen eingesetzte Milchsäure war 80%ig (Wasseranteil 20%). Die Kolonne wurde vor dem Aufstart mit Isopropanol gefüllt.

Man erkennt, daß die Verlängerung der Reaktivzone um eine katalytische Packung fast eine Verdopplung des Durchsatzes (140 statt 75 g/h Milchsäure) bei Fast gleicher Acidity des Sumpfes ermöglicht. Der Isopropyllactatgehalt im Sumpf betrug zwischen 76 und 85%. Das abgezogene Kopfprodukt enthielt Diisopropylether, der als Nebenprodukt durch Veretherung von Isopropanol gebildet wird.

Die Sümpfe der Reaktivdestillation wurden in einer Füllkörper-Kolonne aufdestilliert. Typischerweise wurden folgende Mengen erhalten:
1. 16,5% Vorlauf - Isopropanol 99,8%
2. 1,0% Zwischenlauf - 40% Isopropanol und 60% Isopropyllactat
3. 64,3% Isopropyllactat mit einer Reinheit von 99,65%,
   0,11% Wasser und einer Acidity von 0,07 mg KOH/g
4. 15,3% Restsumpf mit ca. 50% Isopropyllactat, ca. 35% Dilactid und einer Acidity von 59 mg KOH/g.

Der Restsumpf 4 wurde problemlos in der Reaktivdestillation als Edukt wieder eingesetzt.

## Patentansprüche

1. Verfahren zum Herstellen von Estern einer Hydroxysäure und eines C₁-C₈-Alkohols, **dadurch gekennzeichnet, daß** die Veresterung durch Reaktivdestillation an einem heterogenen Katalysator erfolgt.

2. Verfahren nach Anspruch 1, **dadurch gekennzeichnet, daß** die Hydroxysäure eine α-Hydroxysäure ist.

3. Verfahren nach Anspruch 2, **dadurch gekennzeichnet, daß** die α-Hydroxysäure Milchsäure ist.

4. Verfahren nach einem der Ansprüche 1 bis 3, **dadurch gekennzeichnet, daß** der Alkohol ein C₂-C₈-Alkohol ist.

5. Verfahren nach Anspruch 4, **dadurch gekennzeichnet, daß** der Alkohol ein C₂-C₄-Alkohol ist.

6. Verfahren nach einem der Ansprüche 1 bis 5, **dadurch gekennzeichnet, daß** der Alkohol ein primärer oder sekundärer Alkohol ist.

7. Verfahren nach Anspruch 6, **dadurch gekennzeichnet, daß** der Alkohol ausgewählt ist aus der Gruppe bestehend aus Ethanol, n-Propanol, Isopropanol, n-Butanol, 2-Butanol und sec-Butanol.

8. Verfahren nach einem der Ansprüche 1 bis 7, **dadurch gekennzeichnet, daß** der Katalysator in der Destillationskolonne fixiert ist.

9. Verfahren nach Anspruch 8, **dadurch gekennzeichnet, daß** der Katalysator ein saures Ionenaustauscherharz ist.

10. Verfahren nach Anspruch 8 oder 9, **dadurch gekennzeichnet, daß** das niedriger siedende Ausgangsprodukt unterhalb des Katalysators in die Destillationskolonne eingespeist wird.

11. Verfahren nach einem der Ansprüche 8 bis 10, **dadurch gekennzeichnet, daß** das höher siedende Ausgangsprodukt oberhalb des Katalysators in die Destillationskolonne eingespeist wird.

12. Verfahren nach einem der Ansprüche 1 bis 11, **dadurch gekennzeichnet, daß** in den Ausgangsprodukten enthaltenes Wasser sowie Reaktionswasser mittels eines Schleppmittels abgetrennt wird.

13. Verfahren nach einem der Ansprüche 1 bis 12, **dadurch gekennzeichnet, daß** die Reaktionstemperatur bei 60 - 200°C, vorzugsweise 60 - 150°C, weiter vorzugsweise 70 - 100°C liegt.

14. Verfahren nach einem der Ansprüche 1 bis 13, **dadurch gekennzeichnet, daß** ein Lösungsmittel zur Senkung der Sumpftemperatur in den Sumpf geschnitten wird.
